# EUROPEAN PATENT APPLICATION

(11) **EP 4 285 875 A1**
(43) Date of publication of application: **06.12.2023**
(21) Application number: 22315116.8
(22) Date of filing: 02.06.2022
(51) Int. Cl.: A61F 5/00, A61B 17/00, A61B 17/08

(54) **DEVICE AND METHOD FOR PARTITIONING AN INTERNAL TISSUE**

(71) Applicant: Caranx Medical SAS, 75013 Paris (FR)
(72) Inventor: SEJOR, Eric, 06000 Nice (FR); SMITS, Jonas Victor Hamen, 3360 Bierbeek (BE)
(74) Representative: Hepp Wenger Ryffel AG

(57) **Abstract**

A device (10) configured to form a gastric restriction by partitioning the stomach, into at least two compartments. The device comprising an insertion member (11) for insertion within a patient, an implant for partitioning the tissue and first and second fastener portions (22) for securing to first and second opposite tissue walls.

## Description

The present invention relates to a device and method for restricting the size of a cavity in a patient's digestive system. A non-limiting aspect relates to endoscopic creation of gastric restriction, for example, a gastric sleeve.

Gastroenterology is an ever-evolving branch of medicine especially in the field of bariatric disorders for example. This is due in part to the increasing number of obese patients worldwide.

Since the emergence of bariatric surgery in the 1950's leaps and bounds have been made in the field with the apparition of instruments and techniques specifically adapted for gastrointestinal interventions.

In the case of bariatric surgery, gastric bypass and sleeve gastrectomy procedures have proven to provide many advantages over other interventions allowing rapid weight loss but also helping to tackle other issues linked to obesity, for example, diabetes and cardiovascular and respiratory disorders. However, major surgery is expensive and highly invasive, and not suitable for many patients.

The apparition of minimally invasive instruments has further helped progress in the field of bariatric surgery which may be performed laparoscopically or endoscopically. A minimally invasive approach provides the advantages of being less traumatic on the patient's body allowing for faster recovery time, decreased infection rates, as well as taking less operating time thus also being advantageous' for the medical practitioner.

Although minimally invasive instruments as proposed do present advantages, they also present drawbacks. For example, a laparoscopic intervention requires extensive equipment with multiple instruments, and multiple access sites into the body, and can be extremely costly. Also, a laparoscopic approach may not be suitable in some cases, for example, in the case of problematic scar tissue from previous operations.

Endoscopic procedures have the potential to further reduce patient trauma, but current proposals are limited by the size and versatility of endoscopic instruments, for example endoscopic staplers. Such instruments are generally restricted in motion and only operate in a very local manner, creating only small segments of operated tissue. Attempting to create a structure resembling a gastric sleeve can only be achieved by multiple insertions of the instrument, and repetitions in a complicated pattern where tissue portions lie in close proximity. Such a procedure is time consuming, and the degree of success can depend greatly on the skill of the operator.

Some endoscopic devices are used to deploy an inflatable balloon into the stomach, that treats obesity by occupying stomach volume and hence reducing the volume available for food. However, such inflatable devices do not mimic the successful technique of gastric sleeve creation, and care is needed to address correct inflation, and to manage or prevent accidental deflation.

Some devices introducible endoscopically are auxiliary tools for use in a non-endoscopic procedure. US-A-2014/018722 describes an endoscopic device that is positioned in a hollow viscus organ, such as the stomach, and functions as a template for creating a lumen of desired shape and size in the organ. However, the endoscopic device is merely an internal template or support for enabling the lumen to be formed around the template from outside the organ, for example by applying securing sutures from outside the organ, and thus non-endoscopically.

It would be desirable to address one or more of the above issues, to facilitate an endoscopic procedure for restricting the size of a cavity in a patient's digestive system, and optionally to facilitate creation of a gastric restriction, for example, a gastric sleeve.

Aspects of the invention are defined in the claims.

Some aspects of the invention are defined in terms of a device configured to restrict a cavity in a patient's digestive system from within the cavity. The term "device" covers at least an implant that remains in the cavity after deployment by a suitable deployment apparatus, and in which all of the device features are comprised in the implant. The term "device" also covers a combination of at least one implantable element and associated deployment apparatus being used together as a combined device or unit, whether or not all of the features remain in the cavity after deployment. For example, some features of the "device" may belong to the deployment apparatus, and not remain in the cavity after deployment. The term "device" also covers apparatus operable to manipulate tissue of the cavity to create a restriction, whether or not any of the features remain in the cavity after the procedure to create the restriction.

In all cases, the cavity of the digestive system may be the stomach, and optionally the device can create a gastric sleeve made, preferably at least partly, of stomach tissue.

In accordance with a first aspect, the invention provides a device configured to restrict a cavity in a patient's digestive system from within the cavity.

The device is insertable at least partly into the cavity, for example, endoscopically. The device comprises first and second fastener portions for fastening, from within the cavity, to first and second tissue walls of the cavity. The device has at least one operative, spaced configuration of the first and second fastener portions for fastening to respective regions of the first and second walls that are spaced apart on opposite sides of the cavity. Optionally, the first and second fastener portions collectively form a fastener of the device.

Optionally, the device further comprises a restricting element connecting or connectible to the fastener and/or to tissue in the vicinity of the fastener, to operatively restrict the cavity.

At least the restricting element may remain in the cavity as an implanted element after deployment. Optionally, the fastening portions are also part of an implant in combination with the restricting element, or the fastener may at least partly be part of the deployment apparatus.

In either case, by providing first and second fastener portions that can fasten to respective regions of the tissue walls that are spaced apart from each other, the device can engage both tissue walls without relying on having to bring the tissue walls firstly into close proximity with each other. For example, in the spaced configuration, the first and second fastener portions can be brought into fastening engagement with the walls in a natural state of the walls and/or can be brought into fastening engagement with regions of the walls that are remote from a mutual peripheral edge. This can greatly simplify creation of the restriction, allowing easier and/or more versatile positioning of the device, and reduce the number of operations necessary.

It can also facilitate the device being operated remotely and/or autonomously, for example, via robotic manipulation or autonomous software control. Manipulations may be effected using a principal instrument, optionally just a single instrument, inserted through the natural body opening. The first and second fastener portions may be configured to secure to internal tissue along first and second elongate lines of attachment, respectively. By using elongate lines of attachment, the fastener is not limited only to fastening tissue in very local segments, but can extend in a longitudinal direction on both tissue walls. This can enable a relatively long restriction length to be created in a single and/or simple procedure.

At least a portion of the device may be expandable from a low-profile delivery configuration to a tissue engagement configuration defining the spaced configuration of the fastener portions. In some embodiments, the device is self-expandable from the delivery configuration to the engagement configuration. A self-expandable device can be easily deployable and self-conforming to the interior shape of the cavity. Additionally or alternatively, the device may be forcibly expanded from the delivery configuration and/or to the engagement configuration. For example, a deployment balloon may be inflated to generate expansion into contact with the first and second portions of the opposite tissue walls. Use of forcible expansion, such as by a deployment balloon, can if desired assist in positive location of the device with respect to the cavity. For example, the balloon may self-align with the profile of the cavity.

When the device is an implant, the implant may be installed as a unitary device, or as multiple components that are coupled together in situ within the cavity to complete an implant assembly.

Various types of fasteners are envisaged for fastening to tissue from within the cavity. For example, at least one of the fastener portions may comprises a suction or suction-applying device, and/or tissue penetrating fixings (for example staples), and/or a tissue adhesive. The fastener may fasten at least temporarily to tissue (e.g. during deployment), and/or more permanently (e.g. for maintaining the restriction after deployment). Multiple types of fastener may be used together, for example, a first type of fastener (e.g. a suction device) for attaching temporarily to tissue, and a second type of fastener (e.g. a tissue penetrating fixing) for more permanent attachment.

The device may comprise, or further comprise, a first frame portion carrying or comprising the first fastener portion, and a second frame portion carrying or comprising the second fastener portion. Each frame portion may comprise a respective frame member, e.g. optionally a rail. At least one frame portion may be elongate, optionally both frame portions being elongate. Such an arrangement can provide a structure that can engage tissue walls along substantial length, and provide structural integrity independent of, or in addition to, the tissue walls. The frame portions can distribute operational forces along the cavity wall. Additionally or alternatively, the frame portions may provide support for other components of the restriction device, for example as described below.

In some embodiments, the frame portions (or at least one frame portion) are dimensioned to match at least a majority of a length of a stomach. For example, at least one frame member has a length of between about 100mm and 400 mm, optionally wherein both frame members have a length of between about 100 mm and 400mm. Further optionally, at least one frame member or both frame members may have a length between: 100mm-300mm; 100mm-200mm; 200mm-400mm.

In some embodiments, each fastener portion is distributed along the respective frame portion.

In some embodiments, the first and second frame portions are coupled together, for example, at a frame apex.

At least a portion of the device may be collapsible from the tissue engagement configuration to a narrow configuration for establishing the restriction. The restriction device may function to collapse the device to the narrow configuration.

In some embodiments, the device (e.g. the restricting element) may comprise a collapser for drawing first and second portions of the device towards one another to constrict open space between the first and second portions and/or for holding the portions in a narrowed configuration in which open space between the first and second portions is constricted. The collapser may act to substantially close off open space.

The first and second portions may be the first and second frame portions, respectively.

By drawing the device to a narrow, or closed, configuration, the device can bring the opposite walls of the cavity into close proximity, or into a closed relation (e.g. sealed relation) along the length of the implant. This can create a sleeve-like form in the cavity, (e.g. along the length of the implant), for example, in a single operation.

In some embodiments, the collapser comprises a flexible filament coupled to the first and second portions such that tensioning the filament draws the first and second portions towards each other and/or holds the first and second portions in a narrowed configuration. The filament may be laced through apertures distributed along each portion, and optionally criss-cross between the portions. Such a collapser can provide distributed collapsing forces between the frame portions, controlled by tension. The collapser can also be used to control expansion of a self-expanding device, by retaining the device collapsed when under tension, and allowing expansion by relaxing tension.

Optionally, the filament may be configured to interact with the fastener portions. This can permit the frame portions to be made of degradable or resorbable material, while leaving the filament operatively coupled to the fastener portions to maintain the restriction in the cavity. Additionally, or alternatively, the filament may be configured to directly engage or pass through the wall tissue. This also can permit the frame portions and/or the fastener portions to be made of degradable or resorbable material.

Alternatively, in some embodiments, the first and second portions each comprise rails, and the collapser comprises an interlocking element having an interlocking profile slidable along the rails. For example, the interlocking element may have the form of a sheath slidable progressively along the rails (e.g. like a zipper) to progressively bring the rails into, and/or retain the rails in, close proximity to'each other.

Additionally or alternatively to any of the above, the restricting element may comprise a curtain, e.g. a membrane, disposed between the frame portions and/or the fastener portions. The curtain functions to partition the cavity into at least regions or compartments.

Preferably, the restricting element is non-inflating. As used herein, the term "non-inflating" means that the restricting element does not remain substantially inflated in the cavity after completion of deployment, whether or not inflation is used during deployment.

A closely related second aspect provides apparatus for restricting a cavity of a patient's digestive system (for example, in a stomach), the cavity having first and second spaced apart tissue walls on opposite sides of the cavity, the apparatus comprising:
an insertion device insertable through a patient's natural body orifice and into the cavity;
first and second fastener portions for fastening, from within the cavity, to regions of first and second tissue walls of the cavity that are spaced apart on opposite sides of the cavity;
a displacement device for causing the first and second fastener portions to expand from a low-profile delivery configuration to a spaced configuration for engaging said regions of tissue walls on opposite sides of the cavity, and/or for causing the first and second fastener portions to collapse from the or a spaced configuration towards a collapsed configuration to draw the first and second tissue walls towards each other.

At least a part of the apparatus may be defined by an implant that is, during implantation, fastened to the first and second tissue walls by the first and second fastener portions. Additionally or alternatively, at least a part of the apparatus may be removed from the body following creation of the restriction, for example, an instrument or tool for creating the restriction.

The apparatus may include any of the features of the first aspect described above.

A closely related third aspect provides a method of forming a restriction in a cavity of a patient's digestive system (for example, in a stomach), optionally using a device as defined in any of the above aspects, the method comprising:
a. Inserting first and second fastener portions into the cavity through a natural body opening, in a low-profile delivery configuration;
b. Expanding the fastener portions into engagement with regions of first and second tissue walls of the cavity that are spaced apart on opposite sides of the cavity;
c. Fastening the fastener portions to said regions of the first and second tissue walls on opposite sides of the cavity; and
d. Using the first and second fastener portions to form the cavity restriction.

The step of using may, for example, comprise drawing or collapsing the fastener portions towards each other, in order to draw the first and second tissue walls closer together, optionally to narrow or close a gap between the first and second tissue walls. Alternatively, the step of using may comprise using the fastener portions to support a curtain or membrane that partitions the cavity at least partly into first and second compartments, thereby forming a functional restriction.

The method may optionally be carried out under remote control and/or autonomously, for example, via robotic manipulation or autonomous software control. Manipulations in accordance with the method may be effected using a principal instrument, optionally just a single instrument, inserted through the natural body opening.

Non-limiting embodiments of the invention are now described, by way of example only, with reference to the accompanying drawings, in which:
Fig. 1 is a schematic perspective view of a device for partitioning an internal tissue.
Fig. 2 is a schematic perspective view of an alternative embodiment of a device for partitioning an internal tissue.
Figs. 3a-b are schematic views of an alternative embodiment of a device for partitioning an internal tissue.
Figs. 4a-i are schematic illustrations of a method for partitioning an internal tissue according to the embodiments of the device shown in figs. 1-3.
Figs. 5a-b are schematic perspective views of an alternative embodiment of a device for partitioning an internal tissue.
Fig. 6 is a schematic perspective view of a implant of a device for partitioning an internal tissue.
Fig. 7 is a schematic perspective view of an alternative embodiment of a implant of a device for partitioning an internal tissue
Fig. 8 is a schematic perspective view of an alternative embodiment of a implant seen in fig. 7.
Fig. 9 is a schematic perspective view of an alternative embodiment of a implant seen in figs 7 and 8.
Fig. 10a is a schematic cross-section view of an alternative embodiment of a device (shown in fig. 5) for partitioning an internal tissue.
Fig. 10b is a schematic view of an alternative embodiment of a implant in an engaged configuration.
Figs. 11a-h are schematic illustrations of a method for partitioning an internal tissue according to the embodiments of the device shown in figs. 5-10.

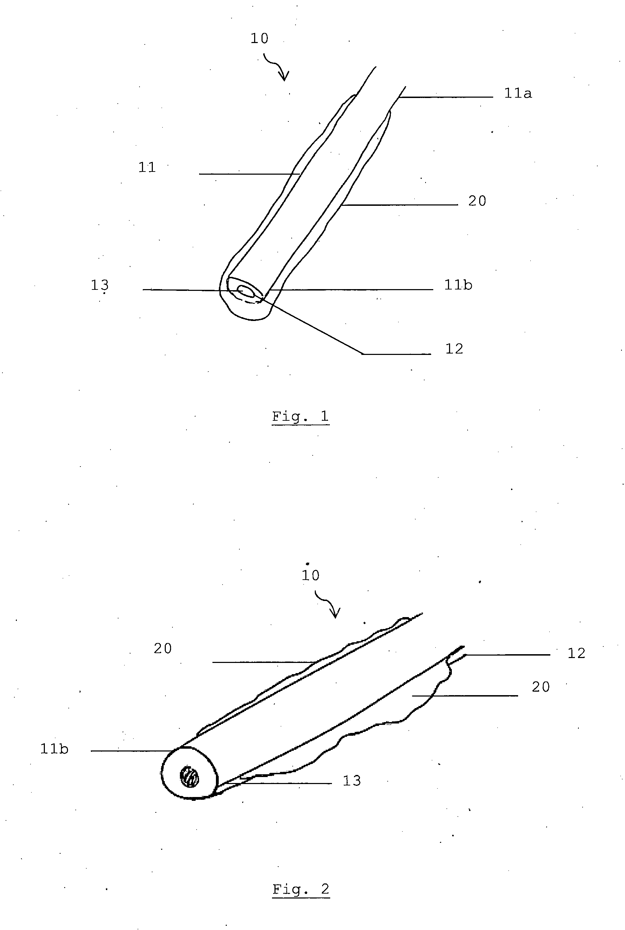

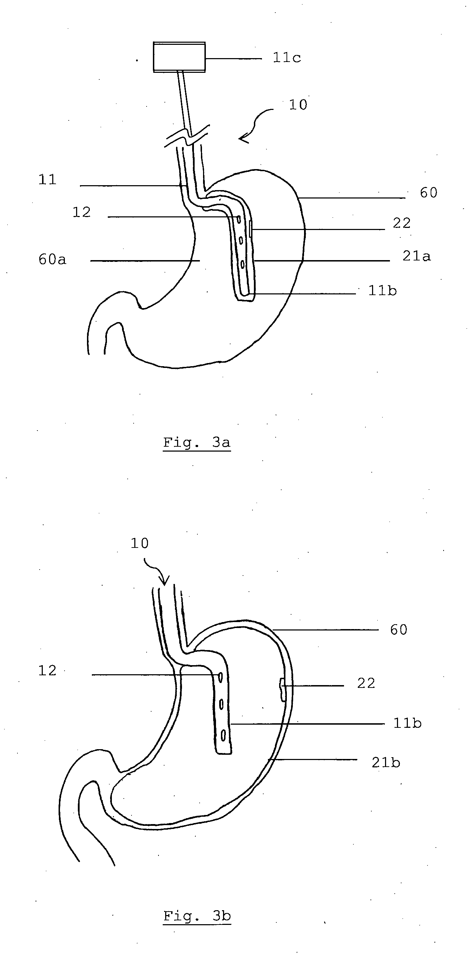

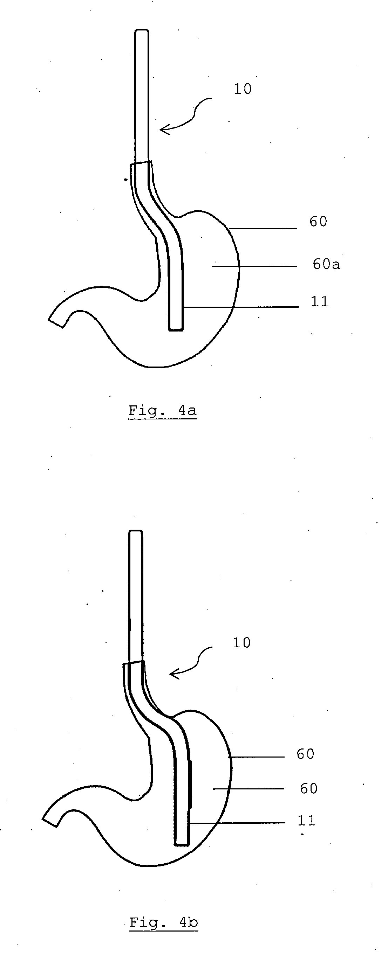

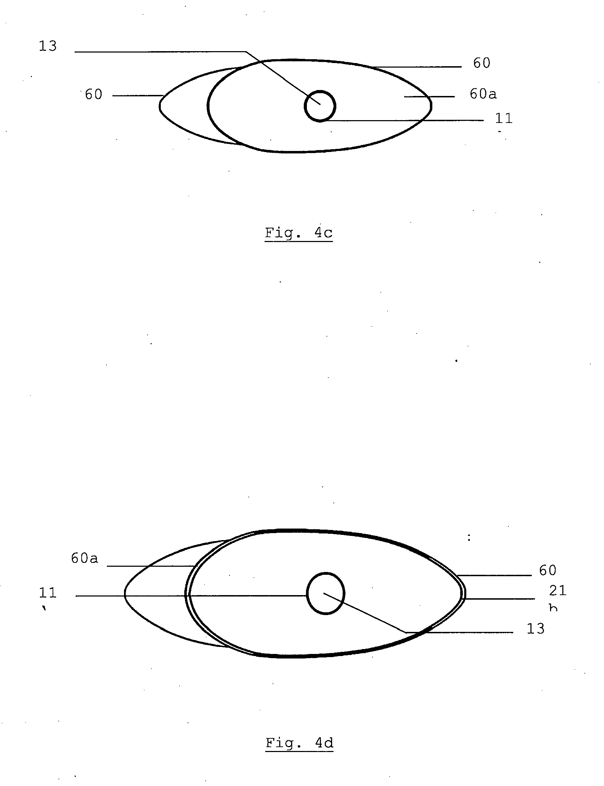

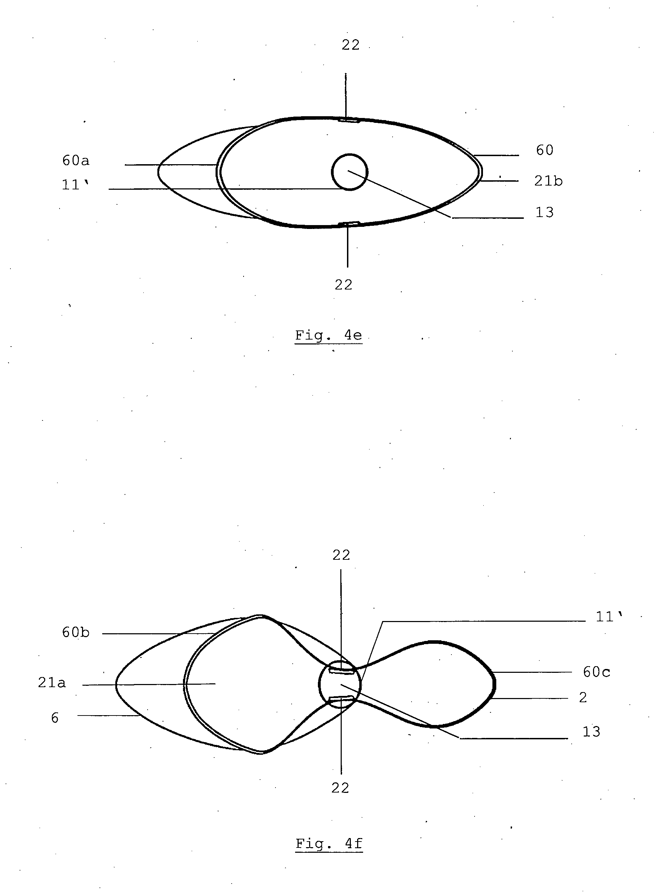

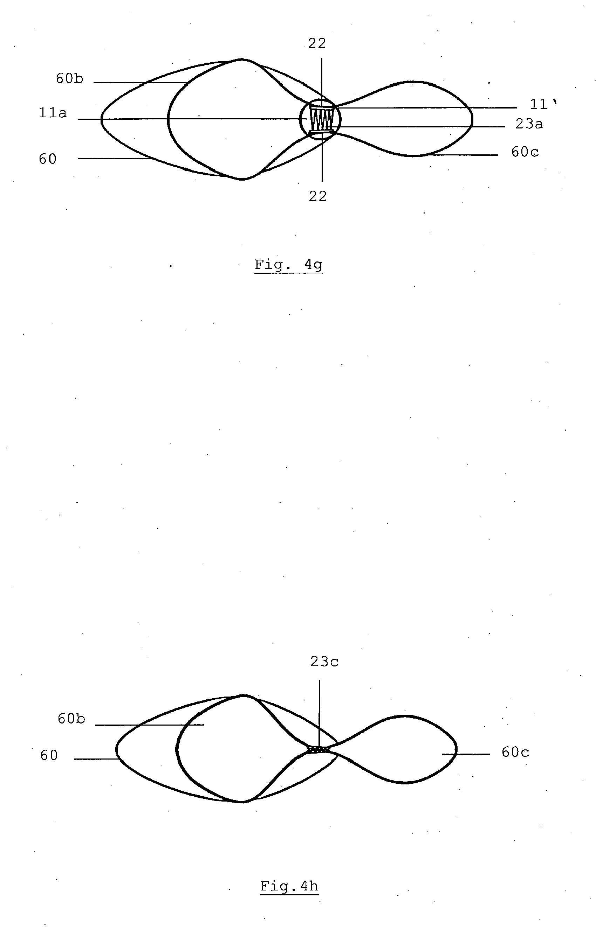

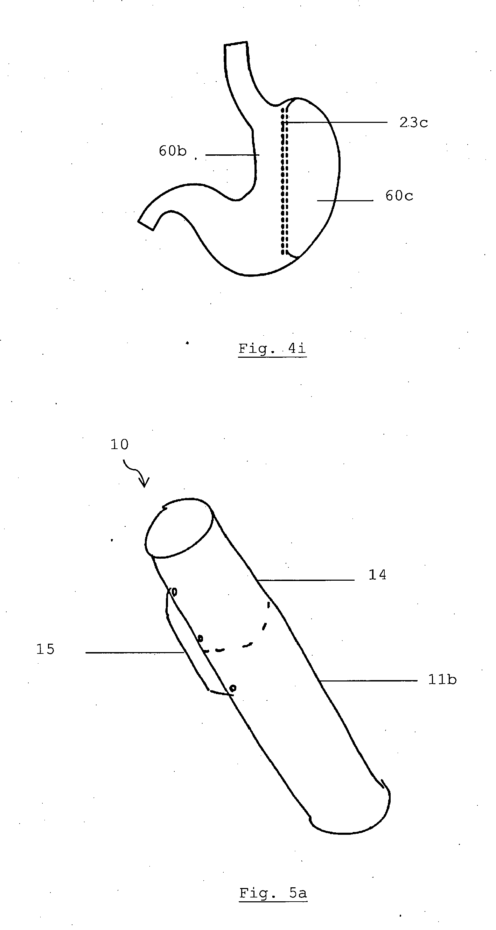

Referring to figs. 1 to 4, a device 10 comprises an insertion member 11 configured for insertion within a patient and further configured for creating a restriction in a natural cavity defined by tissue walls, for example, by partitioning an internal tissue 60 into at least two compartments thus altering the initial functional size 60a (fig. 4) of an internal tissue 60 (figs. 3a and 4) to a reduced functional size 60b (fig. 4f). The internal tissue 60 may be, for example, an organ, preferably an organ of the digestive system, such as an organ of the gastrointestinal tract, for example, a stomach.

One of the principles illustrated in the embodiments is use of first and second fastener portions 22, e.g. carried by the insertion member, to fasten to regions of tissue on first and second walls of the cavity that are spaced apart from each other.

A partition is then formed either by drawing the fastener portions, and hence the spaced part wall regions, towards each other, or by providing an artificial curtain between the faster portions.

The device 10 is configured to be at least partly inserted within a patient. The device 10 is optionally configured for insertion within a patient through a natural body opening, for example, through the mouth of a patient.

The device 10 carries or comprises an implant and/or expandable component 20 (for example, carrying fastener portions '22 described later) and configured to interact with the interior surface of an internal tissue, for partitioning the internal tissue 60.

The device 10 comprises a working end situated at, or near, a distal end 11b of the insertion member 11 and an operator end situated at, or near, a proximal end 11a of the insertion member 11. The operator end provides an operator unit 11c (see fig. 1.a) for operating the working end.

Operation.of the operator unit 11c induces one or more of: positioning of an insertion member 11 with regards to internal tissue; interaction between implant and/or expandable component 20 and an internal tissue; partitioning an internal tissue 60.

The insertion member 11 comprises one or more openings 12 on the insertion member 11, and/or one or more channels 13 extending at least a part of the length of, the insertion member 11. The openings 12 and/or channels 13 are configured to facilitate the partitioning of an internal structure, optionally by facilitating the attaching of the implant to at least a section of an internal tissue 60.

In Fig. 1, the implant and/or expandable component 20 is configured to at least partly circumscribe the insertion member 11.

In Fig. 2, the implant and/or expandable component 20 is configured to deploy at least partly from within a region of the insertion member.11. One or more channels 13 or compartments of the insertion member 11 are configured to at least partly accommodate the implant and/or expandable component 20 (see fig. 2, 5b and 10 a), for example, in a low-profile delivery configuration.

Referring to figs. 3a and 3b, one or more openings 12 of the insertion member 11 can be configured to transmit a negative and/or positive pressure to an expandable component. For example, such openings 12 transmit a positive pressure for changing the expandable component 21 from a non-expanded state 21a to an expanded configuration, optionally by inflating the expandable component 21. The openings 12 may transmit a negative pressure for changing the expandable component 21 from an extended configuration to a non-extended configuration, optionally by deflating the expandable component 21.

Referring to figs. 4a and 4b, the insertion member 11 can also be configured to change configuration for attaching to one or more sections of an internal tissue 60. The insertion member 11 comprises a first insertion configuration for insertion within a patient (see fig. 4a) and a second working configuration for partitioning an internal tissue 23c. The insertion member 11 is configured to adapt from an insertion configuration to a repositioned working configuration 11' (see figs. 3a-b, 4b and 11b-c). The working configuration 11'of the insertion member 11 may be at least partly out-of-line with regards to the insertional configuration.

As will be seen in figs. 4b-g, adapting the configuration of the insertion member 11 places the insertion member 11 in an at' least partly central position with regards to the internal tissue 60.. The at least partly central position of the insertion member 11 is at least partly out-of-line with regards to the insertion configuration. Adapting the configuration of an insertion member 11 may facilitate interaction of an expandable component 21 with an internal tissue 60 of a patient. For example, the insertion member 11 can be configured to at least partially rotate for positioning an expandable component 21 with regards to the target section of internal tissue 60. Alternatively, the insertion member 11 may be displaced (e.g. passively) to a central position as a result of expansion of the expandable component 21.

In the illustrated example, the expandable component 21 comprises a balloon or one or more panels of an at least partly expandable material defining an interior cavity. The expandable component 21 is configured to inflate or expand to an expanded state 21b and/or to deflate or contract to a non-expanded state 21a.

In the expanded state, the expandable component 21 can interact with first and second walls of the cavity, on opposite sides of the cavity. Typically the first wall is the anterior wall and the second wall is the posterior wall. For example, the expandable component 21 temporarily adopts the general shape of the cavity. The expandable component 21 is configured to attach to an internal tissue 60 in an expanded state 21b. One or more fastener portions 22, optionally situated on its external surface, are configured to attach at least temporarily to one or more sections of an internal tissue 60, for example, by being pushed against the tissue by the expandable component 21 and/or by operation of the fastener to more intimately draw the tissue against the fastener.

The one or more fastener portions 22 extend at least a part of the length and/or width of the expandable component. The fastener portion(s) 22 may attach to the internal tissue 60 by any means suitable, for example, by means of a biocompatible adhesive, or one or more gripping (e.g. by means of suction and/or suture) and/or perforating elements (e.g. staples; hooks) 22a for attaching to an internal tissue. Other possible examples of fastener portion 22 are also described in later embodiments.

In the illustrated form, the fastener portions 22 are configured to attach to one or more longitudinally and/or laterally extending sections of internal tissue 60 for creating an at least partly longitudinal partition 23c of an internal tissue 60.

Deflation or collapsing of the expandable component 21 pulls at least a section, optionally the opposing sections, of internal tissue 60 attached to the expandable component 21, inwardly. In the illustrated form, the expandable component 21 may pull tissue sections at least partly within the fastener portion and/or the insertion member 11.

The expandable component 21 is also configured to pull the distanced sections of an internal tissue 60 at least partly closer together and/or into contact with one another. For example, the two sections of internal tissue 60 are brought closer to one another by the expandable component 21 changing from the expanded state 21b to the non-expanded state 21a, for example, by deflation from the expanded state 21b to the non-expanded state 21a. Attachment of the tissue wall regions to the fastening portions cases the tissue walls to follow the expandable component 21 and be drawn inwardly.

The inward pulling or collapsing of the two sections of tissue creates a partition 23c of the organ into a first compartment 60b that will be the new functional compartment of the organ and a second compartment 60c that will no longer be able to receive food, thereby defining a gastric sleeve shape in the stomach cavity.

Depending on the design implementation, the fastener portion 22 can be configured to detach from the expandable component 21 and remain attached to the partition 23c (see fig. 4f-g). Alternatively, the fastener portion 22 (e.g. if made from bioresorbable material) can be at least partly temporary and may be configured to dissolve at least partly over time.

In fig. 4g-h, the fastener portion(s) 22 provides one or more joining features 23 configured to at least partly connect and/or secure one or more fastener portions.

As illustrated below, the joining feature 23 can also be configured to connect and/or bring at least two portions of a fastener at least partly closer to one another and/or to secure the fastener portions 22 with regards the tissue.

Optionally at least two fastener portions 22 comprise one or more joining features 23 configured to connect the at least two fastener portions 22 with regards to one another. The securing of at least a first fastener portion 22 to at least a second fastener portion 22 allows the partition 23c to remain in place. The placing of the joining features 23 and the fastener portion 22 are removable thus allowing the partition 23c to be removed if necessary in the future. The joining feature 23 may be, for example, a suture. The joining feature 23 may be configured to extend at least a part of the length and/or width of a fastener portion. Optionally a plurality of joining features 23 may be at least partly adjacent with regards to one another.

If desired, the joining feature 23, or fastener portions, may be further configured at least partly join one or more fastener portions 22 to one or more sections of an internal tissue 60.

Depicted in fig. 4h, the organ is partitioned into at least two compartments wherein a first compartment 60b is the new reduced functional compartment and a second isolated compartment.

Referring to Fig. 4i, the fastener portion 22 can be configured to secure the partition 23c along an at least partly longitudinally extending section of the organ.

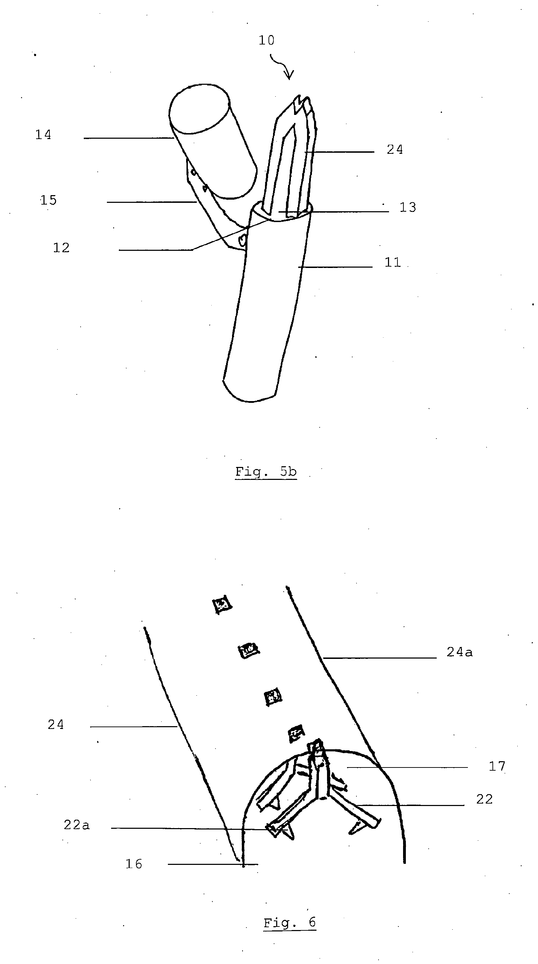

Referring to figs. 5 to 10, in other embodiments the device comprises an implant 20 having one or more frame portions, also referred to herein as rail components 24. The rail components are configured to engage with and or pull one or more spaced regions of internal tissue 60 for forming a partition 23c.

Illustrated in figs. 5a-b, the insertion member 11 can comprise a cover member 14 configured to articulate and/or detach at least partly from the insertion member 11 for allowing the delivery of one or more implants 20.

The cover member 14 is situated towards an extremity of the insertion member 11, optionally at, or near, a distal end 11a of the insertion member 11. The cover member 14 is configured to at least partly detach from an insertion member 11 for delivering and/or positioning one or more implant 20 within an internal tissue 60. In a closed position, the cover member 14 may cover or close the insertion member 11. The cover member 14 may displace to a different axis from the remainder of the insertion member 11.

As seen in figs. 5a-b, in some embodiments the insertion member 11 further comprises a hinged attachment 15 or a hinge for connecting the cover member 14 to the insertion member 11. The hinged attachment 15 allows the cover member 14 to pivot from an aligned position with regards to the insertion member 11 to an at least partly out-of-line position with regards to the insertion member 11.

Referring to figs. 5b to 10, in some embodiments the implant 20 comprises one or more frame portions, in the form of rail components 24 configured to engage with and/or pull one or more sections of an internal tissue 60 for forming a partition 23c of an internal tissue 60.

Optionally, the or each rail component 24 is configured to longitudinally advance within the insertion member 11.

The rail component 24 is at least partly expandable from an inactive and/or low-profile and/or non-deployed configuration accommodated within an insertion member 11 and an active and/or deployed and/or spaced configuration for engaging with an internal tissue 60. The rail may be at least partly flexible for expanding, optionally self-expanding.

The rail component 24 is made of any suitable biocompatible ma-' terial. For example, the rail component may be made from a polymer material (e.g. polylactic acid (PLA); polyether ether ketone (PEEK)).Optionally the rail may be at least partly temporary and may be made from an at least partly bioresorbable material (e.g. polyglycolide (PGA); poly(lactic-co-glycolic acid) (PLGA)).

Additionally or alternatively, the rail component may be at least partly made from any medically suitable metal and/or metal alloy (e.g. nitinol).

In the example of fig. 6, the rail component 24 comprises at least one sleeve element 24a for engaging with and/or pulling at least a section of an internal tissue 60. The sleeve element 24a may at least partly extend from a first extremity of the rail component 24 toward a second extremity, and comprise an elongate aperture 16. The aperture 16 and conduit 17 may facilitate engaging with a section of an internal tissue 60 for partitioning the internal tissue 60.

The sleeve element 24a can be temporary. For example, the sleeve element 24a may at least partly dissolve after a given period of time. The sleeve element 24a may be made of an at least partly bioresorbable material. Optionally, the sleeve element 24a is configured to detach from rail component. Alternatively, the sleeve element 24a can be a permanent element of the rail component. In either case, the sleeve element 24a can be made of a biocompatible material.

In some embodiments the conduit 17 is configured to convey a negative and/or positive pressure for engaging, optionally for disengaging with one or more sections of an internal tissue 60 as a fastener portion. For example, negative pressure may be used to fasten to tissue (e.g. temporarily) by suction. Additionally or alternatively, pressure may be used to drive an actuator for operating a fastener portion.

In the illustrated example, the rail component 24 comprises one or more fastener portions 22 configured at attach to one or more sections of an internal tissue 60. The fastener portion 22 comprises one or more gripping elements 22a configured to at least partly perforate or grip internal tissue 60 for attaching to the section of tissue. Optionally pulling a section of internal tissue 60 into a rail component 24 may at least partly insert one or more fastener portions 22, optionally the gripping element 22a within the section of internal tissue 60.

Additionally or alternatively, the fastener portion 22 provides one or more joining features 23. Optionally, the joining feature may be a thread-like element 23a for engaging the fastener portion(s) 22 with a section of an internal tissue, further optionally the thread-like element 23a may be operated by an operator unit 11c of the device.

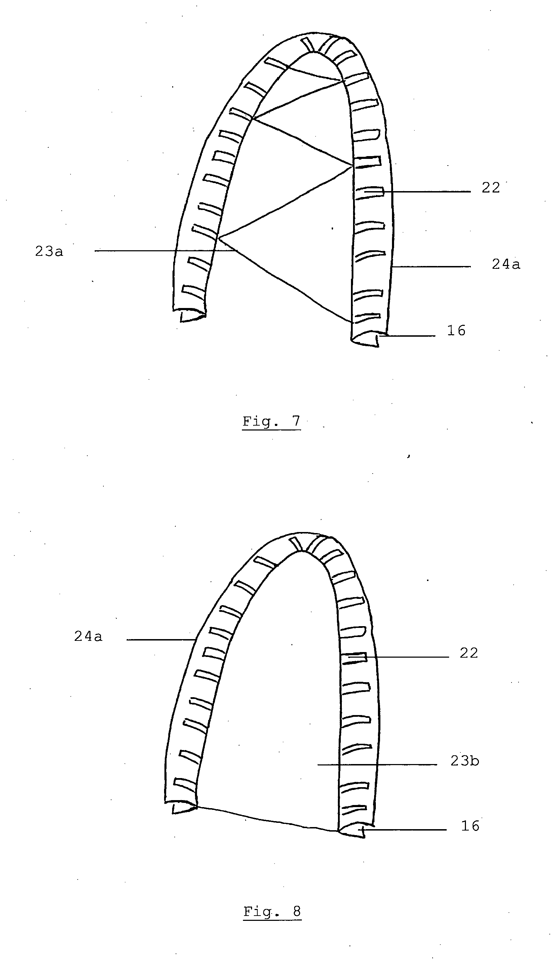

Illustrated in figs. 7 and 8, the sleeve element 24a comprises a joining feature 23 configured to connect two parts of a rail component and/or a plurality of rail components 24. The joining feature 23 may operate to bring at least rail component parts 24 closer to one another. The joining feature 23 may be configured to extend between a first rail component 24 and at least a second rail component 24 in an at least partly linear and/or diagonal manner.

Seen in fig. 8, in some embodiments the joining feature 23 may be an impermeable or at least partly permeable membrane 23b. Optionally, the joining feature 23 may comprise one or more layers of an at least partly permeable membrane 23b for partitioning an internal tissue 23c.

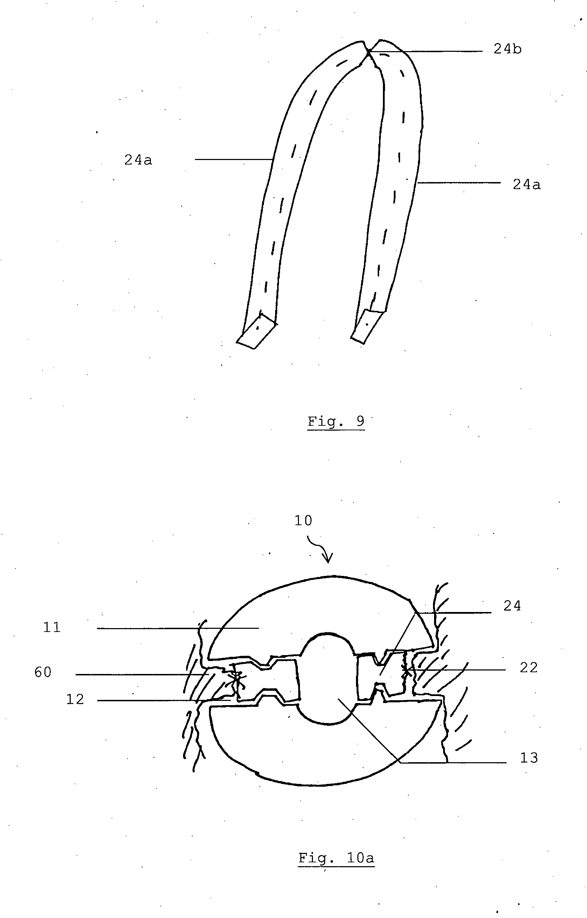

Referring to fig. 9, in some embodiments one or more rail components 24 adopt an at least partly curved shape, for example, a U-shape. The two rail components 24 can have at least one point of contact, optionally at a frame apex.

Illustrated in fig. 10a the insertion member 11 may provide one or more apertures 16 extending at least a part of the length of the insertion member 11 and configured for attaching one or more rails to one or more sections of internal tissue 60 from within an insertion member 11.

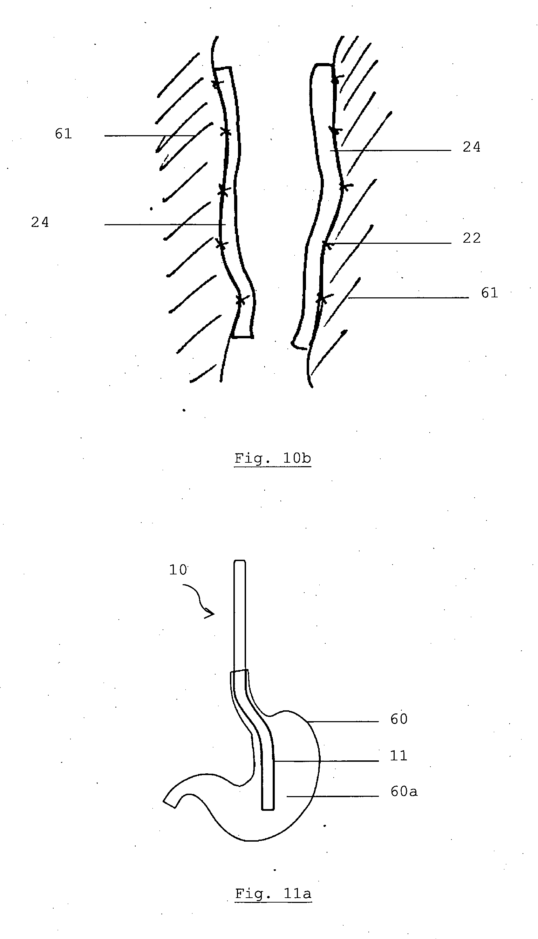

Depicted in figure 10b, in some embodiments two rail components 24 may attach two sections of an internal tissue, optionally attaching the two sections of internal tissue 60 from within an insertion member 11.

Seen in fig. 10b, in some embodiments two rail components 24 may be configured to separately attach to two sections of internal tissue 60.

The two separate rail components 24 may be configured to at least partly attach to one another within an insertion member 11.

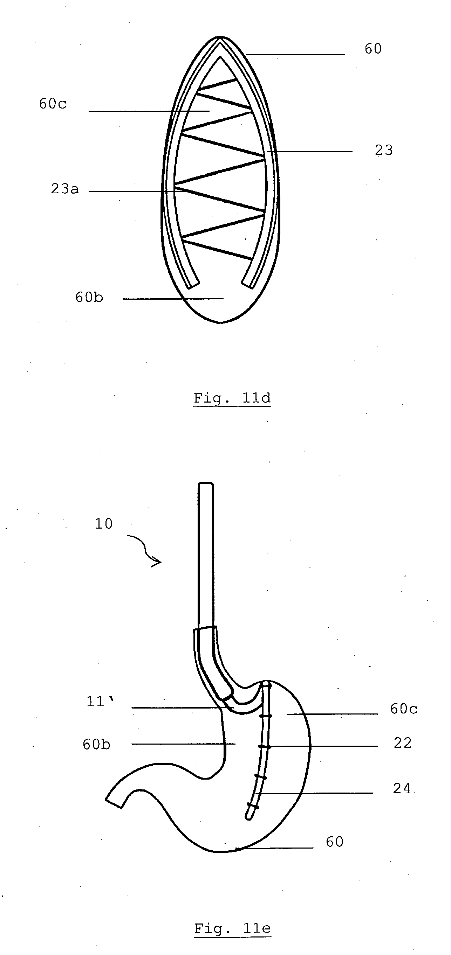

Illustrated in figs. 11a-i is a method for forming a restriction an internal tissue with a device 10 wherein the implant 20 comprises one or more rail components 24.

Referring to fig. 11a the insertion member 11 is inserted within a patient through a natural body opening, for example, the mouth, in an insertion configuration.

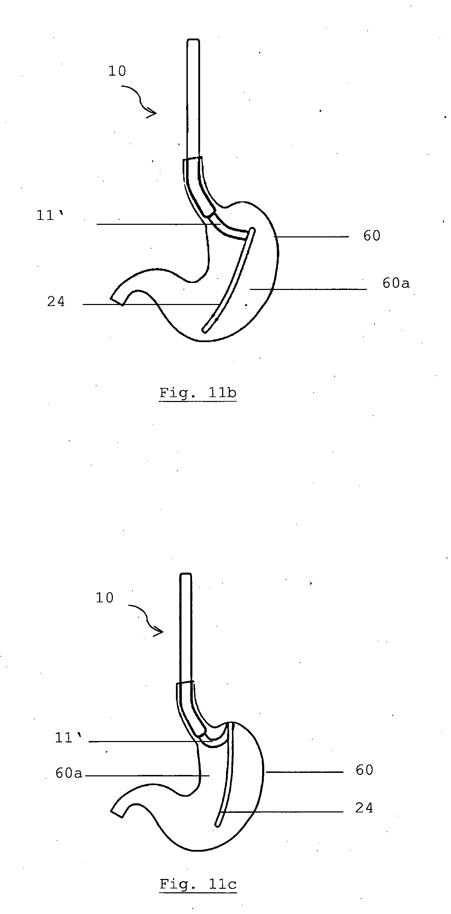

Referring to fig. 11b, the insertion member is adapted to a working configuration 11' wherein a portion of the insertion member 11 is deviated from the insertion configuration. In the working configuration 11' the insertion member delivers one or more rail component 24 to an organ 60, for example, the stomach.

Referring to figs 11c-d, the rail component 24 is positioned within the organ 60. The rail component 24 expands so as to engage with two sections of the organ 60, for example the anterior and posterior wall of the stomach spaced apart on opposite sides of the stomach.

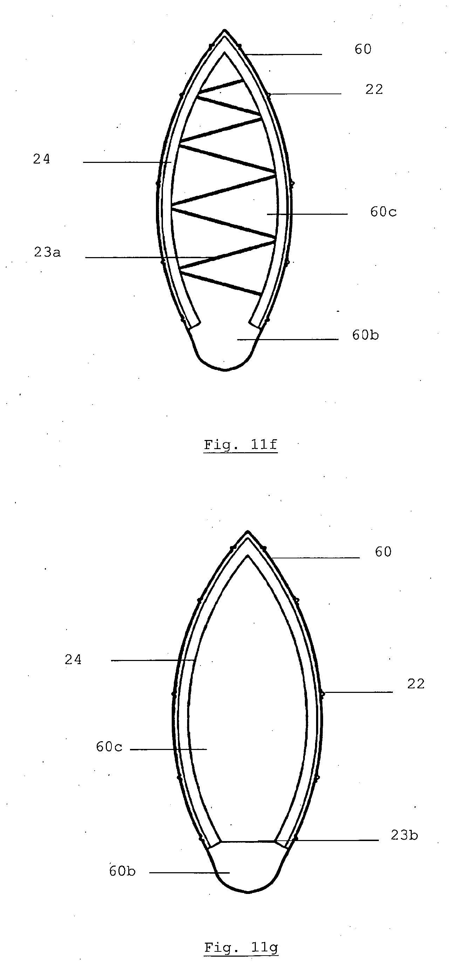

Referring to figs. 11e-g the fastener portions 22 of the rail component 24 engage with the organ wall 60 thus securing the rail component 24 with regards to the organ 60. As seen in 11f, a joining feature 23 connects two parts of the rail component 24 thus joining 23 the two sides of the organ with which the rail component 24 is engaged. The joining feature 23 in fig. 11f is a thread-like element 23a operated so as to bring the two parts of the rail component 24 together (not shown) thus drawing the engaged sections of organ closer to partition the organ, and thus create a functional restriction. Alternatively, in fig. 11g a membrane 23b connects the two parts of the rail component 24 thus creating a barrier between a first compartment 60b and second compartment 60c and therefore partitioning the organ 60 also to create a functional restriction.

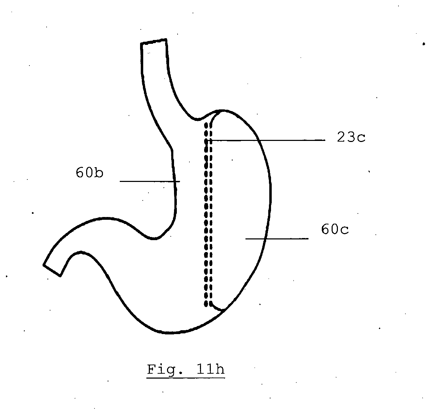

Fig. 11h illustrates a longitudinal partition 23c by means of a rail component 24 can be seen separating an organ into a first compartment 60b and a second compartment 60c. The first compartment 60b is in communication with the gastrointestinal tract and forms the new functional compartment of the organ whilst the second compartment is no longer in communication with the gastrointestinal tract.

It is emphasized that the foregoing description is merely illustrative of example forms of the invention. Many modifications and equivalents may be made within the overall scope of the invention.

## Claims

1. Device (10) configured to restrict a cavity (60) in a patient's digestive system from within the cavity, the device being insertable at least partly into the cavity and comprising first and second fastener portions (22) for fastening, from within the cavity, to first and second tissue walls of the cavity, the device having at least one operative spaced configuration of the first and second fastener portions (22) for fastening to respective regions of the first and second walls that are spaced apart on opposite sides of the cavity.

2. The device according to claim 1, further comprising a restricting element (23, 24) connecting or connectible to the fastener portions, to operatively restrict the cavity.

3. The device according to claim 1 or 2, wherein the device is configured to partition the cavity into first and second compartments (60a, 60b, 60c), each defined at least partly by respective tissue wall portions of the cavity.

4. The device of any preceding claim, wherein the first and second fastener portions (22) are configured to secure the device to internal tissue along first and second elongate lines of attachment, respectively.

5. The device of any preceding claim, wherein at least one of the fastener portions (22) comprises at least one selection, optionally a plurality of selections, from:
a. a suction device or suction-port.
b. tissue penetrating fixings.
c. a tissue adhesive.

6. The device of any preceding claim, wherein the partitioning device comprises a first frame portion (24) carrying or comprising the first fastener portion (22), and a second frame portion (24) carrying or comprising the second fastener portion (22), the first and second frame portions optionally coupled at a frame apex.

7. The device of claim 6, wherein at least one frame portion (24) is elongate, optionally both frame portions (24) being elongate.

8. The device of claim 6 or 7 wherein each fastener portion (22) is distributed along the respective frame portion (24) .

9. The device of any preceding claim, expandable from a low-profile delivery configuration to a tissue engagement configuration, the tissue engagement configuration defining said spaced configuration of the fastener portions (22).

10. The device of any preceding claim, collapsible from the or a tissue engagement configuration defining the spaced configuration of the fasteners, to a narrow configuration for establishing the restriction.

11. The device according to any preceding claim, further comprising a collapser (23) for drawing first and second portions (24) of the device towards one another to constrict open space between the first and second portions (24), and/or for holding the portions (24) in a narrowed configuration in which open spaced between the first and second portions is constricted.

12. The device according to claim 11, wherein the collapser (23) is configured to draw the first and second portions (24) of the partitioning device to substantially close-off open space between the first and second portions (24), and/or for holding the first and second portions (24) in a closed configuration in which open space between the portions is substantially closed-off.

13. The device according to claim 11 or 12, wherein the collapser (23) comprises a flexible filament coupled to the first and second portions (24) such that tensioning the filament draws the first and second portions (24) towards each other and/or holds the first and second portions (24) in a narrowed configuration.

14. The device according to claim 11 or 12, wherein the first and second portions (24) each comprise a rail profile, and wherein the collapser comprises an interlocking element (11) having an interlocking profile slidable along the rail profiles, optionally wherein the interlocking element (11) comprises an interlocking sheath slidable progressively along the rail profiles to progressively bring the rail profiles into, and/or to retain the rail profiles in, close proximity to each other.

15. The device according to any preceding claim, comprising a membrane wall (23b)to define a partition barrier.

16. The device according to any preceding claim, comprising an implant, the implant comprising the first and second fastener portions (22).

17. The device according to any preceding claim, comprising a manipulation instrument, the instrument comprising the first and second fastener portions (22).

18. A method of forming a restriction in a cavity of a patient's digestive system, optionally using a device as defined in any preceding claim, the method comprising:
a. Inserting first and second fastener portions into the cavity through a natural body opening in a low-profile delivery configuration;
b. Expanding the fastener portions into engagement with regions of first and second tissue walls of the cavity that are spaced apart on opposite sides of the cavity;
c. Fastening the fastener portions to said regions of the first and second tissue walls on opposite sides of the cavity;
d. Using the first and second fastener portions to form the cavity restriction.
